Europäisches Patentamt

European Patent Office

Office européen des brevets

(11) Publication number: **0 368 409**
**A2**

# EUROPEAN PATENT APPLICATION

(21) Application number: **89202799.6**

(22) Date of filing: **07.11.89**

(51) Int. Cl.⁵: **A61K 31/485, A61K 47/10,**
**A61K 47/12, A61K 47/14**

(30) Priority: **10.11.88 US 269943**

(43) Date of publication of application:
**16.05.90 Bulletin 90/20**

(84) Designated Contracting States:
**AT BE CH DE ES FR GB GR IT LI LU NL SE**

(71) Applicant: **Norwich Eaton Pharmaceuticals, Inc.**
**17 Eaton Avenue**
**Norwich New York 13815(US)**

(72) Inventor: **Szuktak, Joan Bolduc**
**5244 Pro's Drive**
**West Chester Ohio 45069(US)**
Inventor: **Manring, Gary Lee**
**263 South Gersam Avenue**
**Hamilton Ohio 45013(US)**
Inventor: **Smith, Ronald Lee**
**7588 Devonwood Drive**
**West Chester Ohio 45069(US)**
Inventor: **Drust, Eugene George**
**1 Hillview Drive**
**Norwich New York 13815(US)**

(74) Representative: **Suslic, Lydia et al**
**Procter & Gamble European Technical**
**Center N.V. Temselaan 100**
**B-1820 Strombeek-Bever(BE)**

(54) **Compositions for the transdermal delivery of buprenorphine salts.**

(57) Compositions for the transdermal delivery of buprenorphine salts comprising a safe and effective amount of a pharmaceutically-acceptable buprenorphine salt in a carrier comprising:

(a) a polar solvent material selected from the group consisting of $C_3$-$C_4$ diols, $C_3$-$C_6$ triols, and mixtures thereof; and

(b) a polar lipid material selected from the group consisting of fatty acids, fatty alcohols, fatty alcohol esters, fatty acid esters, and mixtures thereof; wherein said polar solvent material and said polar lipid material are present in a weight ratio of solvent material:lipid material of from about 60:40 to about 99:1. Preferably, the polar solvent material is propylene glycol, and the polar lipid material is a $C_{12}$-$C_{18}$ fatty alcohol, a $C_{12}$-$C_{18}$ fatty acid, or an ester of a $C_8$-$C_{12}$ fatty alcohol or fatty acid. Particularly preferred polar lipid materials include oleic acid, lauryl alcohol, methyl laurate and methyl caprylate. The ratio of polar solvent material to polar lipid material is preferably from about 90:10 to about 98:2.

# COMPOSITIONS FOR THE TRANSDERMAL DELIVERY OF BUPRENORPHINE SALTS

## BACKGROUND OF THE INVENTION

This invention relates to novel compositions for delivery of pharmaceutical active materials through the skin of humans and other animals. In particular, this invention relates to compositions for systemic administration of buprenorphine salts, by topical application.

The pharmaceutical literature describes many methods for systemic delivery of pharmaceutical active materials, i.e., delivery of actives to internal organs via the circulatory system. Typically, such methods are either parenteral (by intravenous or intramuscular injection) or enteral (by oral ingestion). However, methods are also described for delivery of active materials transdermally, i.e., systemic delivery through the skin from a topically applied composition. Such compositions may be applied to the skin as an ointment, for example. Alternatively, many devices are known in the literature which contain a set quantity of the composition in close proximity to the skin allowing the active to contact and penetrate the skin. These devices are commonly referred to as "transdermal patches".

Transdermal administration of pharmaceutical actives may offer many advantages, at least in theory. Transdermal delivery is often more convenient than parenteral or enteral administration, avoiding the need for injections or repeated administration of oral dosages. Therefore, compliance with therapeutic regimens, which is often critical, can be improved. Precise control of the dosage may also be afforded, avoiding potential overdoses. Blood levels of the active may be more stable and uniform (without sharp "peaks"), thereby reducing side effects. Further, transdermal delivery may avoid complications associated with enteral administration of pharmaceutical actives. For example, variable rates of absorption through the gastrointestinal tract, gastrointestinal irritation, and hepatic first-pass metabolism may be avoided.

In reality, however, transdermal administration is difficult or even impossible for certain pharmaceutical actives. The skin is highly impermeable, because it must serve as a barrier to pathogens and toxic materials, while also containing the fluids of the body. Much of this impermeability results from the layers of skin created by normal development and physiological changes.

After cells are formed in the basal layer of the skin, they begin to migrate toward the surface until they are eventually sloughed off. As they undergo this migration, they progressively become more dehydrated and keratinized. When they reach the surface, just prior to being sloughed off, they form a thin layer of dense, metabolically inactive cells approximately 10 microns thick. This layer is called the stratum corneum. As a result of the high degree of keratinization, the cells of the stratum corneum provide a formidable barrier.

Thus, in order to achieve systemic delivery, transdermal administration involves penetration of an active material through the dense, lipophilic layer of keratinized skin, as well as through the hydrated basal layers of the skin, in order to reach the circulatory system. Not surprisingly, the ability to achieve this transdermal delivery may depend upon many factors, including (for example) the molecular size of the pharmaceutical active material, its hydrophilic/lipophilic characteristics, and the presence of other materials applied to the skin along with the active material.

The literature is replete with many formulations designed to achieve transdermal delivery of various active materials. Typically, these formulations optimize delivery of a single material, or a small class of materials. See, for example, R. W. Baker et al., Pharmaceutical Technology 1987 26 (1987). Topical compositions containing a lower alkyl diol and a cell envelope disordering compound, for delivery of a variety of lipophilic active materials, are described in European Patent Publication 43,738, Wickett et al., published January 13, 1982. Similar compositions for the delivery of corticosteroids are described in U.S. Patent 4,552,872, Cooper et al., issued November 12, 1985. Compositions for the transdermal delivery of naloxone, naltrexone and nalbuphine, using polyethylene glycol monolaurate, are described in U.S. Patent 4,573,995, Chen et al., issued March 4, 1986. Compositions for the delivery of actives using higher monoalcohols and various solvents such as thio glycerols and lactic acid esters, are described in U.S. Patent 4,590,190, Saito et al., issued May 20, 1986. Compositions containing a lower alcohol and an adjuvant such as an aliphatic hydrocarbon, or a monohydric alcohol ester of an aliphatic carboxylic acid, are described in U.S. Patent 4,593,048, Sato et al., issued June 3, 1986. Pharmaceutical compositions for the transdermal delivery of opioids, using propylene glycol with fatty alcohols or fatty acids, are described in U.S. Patent 4,626,539, Aungst et al., issued December 2, 1986.

While many such delivery systems are known, they may not be acceptable for delivery of certain active materials, for a variety of reasons. For example, the formulations must provide for sufficient flux of the active material through the skin so as to obtain and maintain adequate systemic delivery. This is particularly important since the available skin surface for application is typically confined, for practical reasons, to less than about 100 cm$^2$ (15.5 in$^2$). Compositions must also provide for delivery of the active material while minimizing side effects such as (for example) skin irritation. Further, compositions must be stable, allowing use after extended periods of storage. They should also be compatible with excipient materials, such as gelling agents, in order to allow tailoring of dosage forms to specific needs.


## SUMMARY OF THE INVENTION

The present invention provides compositions for the transdermal delivery of buprenorphine salts comprising a safe and effective amount of a pharmaceutically-acceptable buprenorphine ssalt in a carrier comprising:

(a) a polar solvent material selected from the group consisting of $C_3$-$C_4$ diols, $C_3$-$C_6$ triols, and mixtures thereof; and

(b) a polar lipid material selected from the group consisting of fatty acids, fatty alcohols, fatty alcohol esters, fatty acid esters, and mixtures thereof; wherein said polar solvent material and said polar lipid material are present in a weight ratio of solvent material:lipid material of from about 60:40 to about 99:1.

Preferably, the polar solvent material is propylene glycol, and the polar lipid material is a $C_{12}$-$C_{18}$ fatty alcohol, a $C_{12}$-$C_{18}$ fatty acid, or an ester of a $C_8$-$C_{12}$ fatty alcohol or fatty acid. The ratio of polar solvent material to polar lipid material is preferably from about 90:10 to about 98:2.

It has now been discovered that buprenorphine may be systemically administered at effective levels by such compositions containing buprenorphine salt in a carrier comprising a lower alkyl diol and a fatty acid, a fatty alcohol, or an ester of a fatty alcohol or fatty acid. In particular, such compositions provide efficacious administration of buprenorphine, at high rates of delivery for extended periods of time, without undue side effects (i.e., for example, having reduced skin irritation). These compositions are stable, aesthetically acceptable, and may be adapted to a variety of dosage forms, including transdermal patches.


## DESCRIPTION OF THE INVENTION

The compositions of the present invention comprise a buprenorphine salt in a carrier of a polar solvent material and a polar lipid material, for administration to a human or other animal subject. Specific materials used in this invention must, accordingly, be pharmaceutically acceptable. As used herein, such a "pharmaceutically acceptable" component is one that is suitable for use with humans and/or animals without undue adverse side effects (such as toxicity, irritation, and allergic response) commensurate with a reasonable benefit/risk ratio. Further, as used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used in the manner of this invention. The specific "safe and effective amount" will, obviously, vary with such factors as the particular condition being treated, the physical condition (including age) of the patient, the duration of treatment, the nature of concurrent therapy (if any), and the specific formulations employed.

Specifically, the compositions of this invention comprise a safe and effective amount of a pharmaceutically-acceptable buprenorphine salt in a carrier comprising:

(a) a polar solvent material selected from the group consisting of $C_3$-$C_4$ diols, $C_3$-$C_6$ triols, and mixtures thereof; and

(b) a polar lipid material selected from the group consisting of fatty acids, fatty alcohols, fatty alcohol esters, fatty acid esters, and mixtures thereof; wherein said polar solvent material and said polar lipid material are present in a weight ratio of solvent material:lipid material of from about 60:40 to about 99:1.

The polar solvent material and polar lipid material are preferably present at a combined level (level of polar solvent plus polar lipid) of from about 70% to about 99.5%, more preferably from about 80% to about 99.5%. (As used herein, all percentages are by weight of total composition.)

The weight ratio of polar solvent material to polar lipid material (polar solvent:polar lipid) is preferably from about 80:20 to about 99:1, more preferably from about 90:10 to about 98:2. These preferred ranges are, however, predicated on maximizing systemic delivery of buprenorphine through the skin. Other ratios of polar solvent:polar lipid may be desired in certain formulations, (for example) to enhance aesthetic characteristics, further reduce skin irritation, or to further increase stability of the formulations.

The useful ranges of polar solvent:polar lipid may subsume two phase systems, depending upon the solubility of the particular lipid material in the particular solvent material. Preferably, however, the compositions of this invention are single phase systems, i.e., wherein the polar lipid material is fully soluble in the polar solvent material, in the final formulation (including the active and all optional materials). Accordingly, for example, in compositions employing propylene glycol as the polar solvent material and methyl laurate as the polar lipid material, the ratio of polar solvent: polar lipid is preferably about 97:3. For compositions employing propylene glycol with methyl caprylate as a polar lipid material, the ratio is preferably from about 90:10 to about 95:5.

The safe and effective amount of buprenorphine salt in the compositions of this invention will depend, of course, on the condition to be treated, the desired flux characteristics of the specific composition used, and the desired duration of buprenorphine delivery from the composition. (As referred to herein, "flux" is the rate at which buprenorphine is transferred from the skin surface to the bloodstream). The compositions of this invention may include from about 1.0 mg (milligrams) to about 40 mg, preferably from about 2.0 mg to about 20 mg, more preferably from about 3.0 mg to about 10 mg of buprenorphine as a buprenorphine salt.

The specific amount of buprenorphine salt used in the compositions of this invention may also vary depending upon the solubility of the buprenorphine salt in the other components of the composition (unless a suspension of buprenorphine is desired). The maximum amount of the buprenorphine salt that may be soluble in the other components of the composition (the saturation level) will vary with the specific polar solvent material, polar lipid material, and optional materials present (such as solubilizers). This level may be determined by routine experimentation. Preferably, the buprenorphine salt is present at a level of at least about 50% of saturation. More preferably, the buprenorphine salt is present at a level of at least about 80%, more preferably at least about 90% of saturation.

The compositions of this invention are preferably provided in unit dosage form. As used herein, a "unit dosage form" is a composition of this invention containing an amount of buprenorphine that is suitable for topical application to the skin of a human or lower animal subject, in a single dose, according to good medical practice. As used herein, "topical" application refers to any method by which the compositions of this invention are placed in direct contact with the skin of a subject, or in indirect contact through a permeable interfacing material, such that the flux of buprenorphine from the patch through the skin is, for an adult (weighing approximately 70 kilograms), at least about 20 $\mu$g (micrograms) /hr (hour), preferably from about 40 $\mu$g/hr to about 100 $\mu$g/hr.

The compositions of this invention are preferably administered by use of a device which contains the composition in close proximity to the skin, i .e., a "transdermal patch". Many such structures are known in the pharmaceutical literature. Such patches preferably apply a composition of this invention to an area of skin totalling from about 1cm$^2$ to about 50cm$^2$ (about 0.16in$^2$ to about 7.75in$^2$), more preferably from about 5cm$^2$ to about 10cm$^2$ (about 0.78in$^2$ to about 1.55in$^2$).

Buprenorphine:

The compositions of this invention contain a safe and effective amount of a pharmaceutically-acceptable buprenorphine salt. As used herein, "buprenorphine" refers to N-cyclopropylmethyl-7$\alpha$-[1 -(S)-hydroxy-1,2,2-trimethylpropyl]6,14-endoethano-6,7,8,14-tetrahydronororipavine, and related compounds described in U.S. Patent 3,433,791, Bentley, issued March 18, 1969 (incorporated by reference herein).

The "buprenorphine salts" useful in the compositions of this invention include any pharmaceutically-acceptable salt formed at the amine group of buprenorphine. Many such salts are known in the art, including those described in the following references, all incorporated by reference herein: S. Berge et al., "Pharmaceutical Salts", 66 J. Pharm. Sci. 1 (1977); P. Gould, "Salt selection for basic drugs", 33 Int. J. of Pharmaceutics 201 (1986); and World Patent Publication 87/05297, Johnston et al., published September 11, 1987. Preferred salts include the hydrohalic acid salts, such as hydrochloric acid.

Preferred buprenorphine salts of this invention also include mixtures of buprenorphine with a pharmaceutically-acceptable acid. In such compositions, the buprenorphine and the pharmaceutically-acceptable acid are mixed in the composition as discrete components. The pharmaceutically-acceptable acid is preferably present in an amount such that the number of equivalents of buprenorphine in the composition is about equal to the number of equivalents of acid in the composition (i.e., the number of moles of acid multiplied by the number of acid groups available on the acid).

Many such pharmaceutically-acceptable acids useful herein are known in the art. Preferred

4

pharmaceutically-acceptable acids include, for example, the hydrohalic acids (such as hydrochloric acid) sulfuric acid, phosphoric acid, and the carboxylic acids (such as acetic acid, propionic acid, citric acid, tartaric acid, malic acid, maleic acid, lactic acid, malonic acid, fumaric acid, salicylic acid, succinic acid, ascorbic acid, benzoic acid, and mixtures thereof. Particularly preferred pharmaceutically-acceptable acids useful herein include citric acid and lactic acid.

Also, in addition to the buprenorphine salt, the compositions of this invention may contain other active materials. Such additional active materials should not, however, substantially interfere with the transdermal delivery of buprenorphine or its desired therapeutic effects.

Polar solvent material:

As used herein, "polar solvent material" refers to $C_3$-$C_4$ diols, $C_3$-$C_6$ triols, and mixtures thereof. Such polar solvent materials include, for example, 1,2-propane diol, 1,3-propane diol, 1,2-butane diol, 1,3-butane diol, 1,4-butane diol, 1,2,6-hexane triol, 1,2,5-pentane triol, 1,2,4-butane triol, 1,2,5-hexane triol, glycerol or mixtures thereof. Preferred polar solvent materials include 1,2-butane diol, 1,2-propane diol, glycerol, and 1,2,6-hexane triol. A particularly preferred polar solvent material is 1,2-propane diol (propylene glycol).

Polar lipid material:

As used herein, "polar lipid material" refers to fatty acids, fatty alcohols, fatty alcohol esters, fatty acid esters, and mixtures thereof. Preferred polar lipid materials have from 6 to 20 carbon atoms having a melting point of less than about 50°C (122°F). Fatty alcohols, fatty acid esters, fatty alcohol esters, and mixtures thereof are preferred fatty lipid materials. Polar lipid materials among those useful herein are described in the following patent documents, incorporated by reference herein: European Patent Publication 43,738, Wickett et al., published January 13, 1982; and U.S. Patent 4,626,539, Aungst et al., issued December 2, 1986.

The fatty acids useful herein include straight-chain or branched-chain fatty acids, preferably having from 8 to 20 carbon atoms in their longest carbon chain. Preferred fatty acids include unsaturated fatty acids having one or two double bonds and from 12 to 18 carbon atoms, and saturated fatty acids having from 8 to 16 carbon atoms. Preferably, the fatty acids are straight-chained.

Fatty acids useful herein include (for example) caprylic acid, capric acid, lauric acid, myristic acid, oleic acid, linolenic acid, and linoleic acid. Preferred fatty acids include lauric acid, oleic acid, and linoleic acid. Oleic acid is a particularly preferred fatty acid.

The fatty alcohols useful herein include straight-chain or branched-chain fatty alcohols, preferably having from 8 to 20 carbon atoms in their longest carbon chain. Preferred fatty alcohols include unsaturated fatty alcohols having one or two double bonds and from 12 to 18 carbon atoms, and saturated fatty alcohols having from 8 to 16 carbon atoms. Preferably, the fatty alcohols are straight-chained.

Fatty alcohols useful herein include (for example) octanol, decanol, lauryl alcohol, myristyl alcohol, myristoleyl alcohol, palmitoleyl alcohol, oleyl alcohol, linolenyl alcohol, linolyl alcohol, elaidyl alcohol, vaccenyl alcohol, petroselinyl alcohol, petroselaidyl alcohol, and mixtures thereof. Preferred fatty alcohols include lauryl alcohol, myristyl alcohol, and oleyl alcohol.

Fatty acid esters and fatty alcohol esters useful herein include monoesters and diesters, containing from 6 to 18 carbon atoms in their longest carbon chain. Preferred monoesters have carbon chains of from 8 to 12 carbon atoms in length. Monoesters include saturated monoesters of a $C_6$-$C_{14}$ acid with a linear or branched $C_1$-$C_6$ alcohol, or of a $C_6$-$C_{14}$ alcohol with a linear or branched $C_2$-$C_6$ acid. Monoesters useful in this invention also include unsaturated monoesters of a $C_6$-$C_{18}$ acid with a linear or branched $C_1$-$C_6$ alcohol, and monoesters of a linear or branched $C_6$-$C_{18}$ alcohol with a $C_2$-$C_6$ acid. Diester polar lipid materials of this invention include diesters of a $C_2$-$C_8$ dicarboxylic acid with a linear or branched $C_1$-$C_6$ alcohol, and diesters of a $C_2$-$C_8$ linear or branched, dihydroxy alcohol with a $C_2$-$C_6$ acid.

Such fatty acid and fatty alcohol esters useful in the compositions of this invention include, for example, methyl caproate, propyl caproate, hexyl acetate, methyl heptylate, pentyl heptylate, heptyl acetate, heptyl caproate, methyl caprylate, propyl caprylate, octyl acetate, octyl butylate, methyl caprate, ethyl caprate, hexyl caprate, methyl pelargonate, butyl pelargonate, lauryl acetate, lauryl butylate, methyl laurate, ethyl laurate, isopropyl laurate, hexyl laurate, $C_{12}$-$C_{15}$ alcohols lactate, methyl myristate, ethyl myristate, isopropyl myristate, pentadecyl acetate, methyl palmitate, ethyl palmitate, isopropyl palmitate, hexadecyl acetate, methyl oleate, ethyl oleate, butyl oleate, dimethyl adipate, diisopropyl adipate, diisobutyl adipate,

5

dimethyl maleate, diisopropyl malate, dibutyl malate, dihexyl maleate, and mixtures thereof. Preferred esters include methyl caprylate, propyl caprylate, octyl acetate, methyl caproate, propyl caproate, methyl laurate, $C_{12}$-$C_{15}$ alcohols lactate, diisopropyl adipate, and diisobutyl adipate. Methyl caprylate, methyl laurate, and mixtures of methyl caproate and methyl caprylate are particularly preferred.

## Optional components:

The compositions of this invention may also contain pharmaceutically acceptable optional components that modify the physical and/or therapeutic effects of the compositions. Such optional components may include, for example, additional solvents, emulsifiers, gelling agents, fragrances, preservatives, and stabilizers. However, such optional materials must not unduly interfere with the transdermal delivery of buprenorphine. Optional components useful in the compositions of this invention are described in the following patent documents, incorporated by reference herein: European Patent Publication 43,738, Wickett et al., published January 13, 1982; and U.S. Patent 4,552,872, Cooper et al., issued November 12, 1985.

A preferred optional material is a solvent or co-solvent material, which enhances the solubility of the polar lipid material and/or buprenorphine in the polar solvent material. Such solvent materials include, for example, short chain alcohols and ethers. Preferred optional solvent materials include ethanol, isopropanol, and dimethyl isosorbide. Such optional solvent materials are particularly preferred when the polar lipid material is poorly soluble in the polar solvent material (e.g., long chain esters having carbon chains of from 14 to 18 carbon atoms in length).

Water may also be used as a solvent or co-solvent in the compositions of this invention. Water is a preferred optional component in compositions comprising a fatty acid or a fatty alcohol in the polar lipid material. The presence of water may adversely affect the compositions when the polar lipid material and/or the buprenorphine is near the level of saturation in the polar solvent material. If water is used in a saturated system, a gel or emulsion is preferably formed.

Thickeners, or gelling agents, are also preferred optional materials useful in the compositions of this invention. Thickeners among those useful herein include particulate thickeners and polymeric thickeners such as guargum, methylcellulose, methylhydroxypropylcellulose, polypropylcellulose, polypropylhydroxyethylcellulose, hydroxyethylcellulose, hydroxypropylcellulose and starches and starch derivatives.

The following non-limiting examples illustrate the compositions, processes and uses of the present invention.

## EXAMPLE I

A transdermal patch is made, according to this invention, for delivery of buprenorphine. The buprenorphine composition comprises:

| Component | % (by weight) |
|---|---|
| buprenorphine HCl | 4.0 |
| propylene glycol | 87.0 |
| methyl caprylate | 8.2 |
| hydroxypropylcellulose NF | 0.80 |

The propylene glycol is added to a flask, and while stirring, the hydroxypropylcellulose is added. The mixture is quickly heated, to approximately 100°C (212°F), dissolving the hydroxypropylcellulose. After dissolution, the mixture is slowly cooled to room temperature (approximately 27°C, 80°F), to minimize vapors.

The methyl caprylate is then added, and stirred for approximately 15 hours, at room temperature. The mixture is then heated, and the buprenorphine HCl added slowly, followed by heating (to approximately 90°C, 194°F) with stirring, to dissolve the buprenorphine. After dissolution, the composition is allowed to cool slowly.

A patch structure is made by placing a sheet of lens tissue measuring approximately 4 cm (centimeters) x 10 cm, on a 5 cm x 12 cm sheet of polymer coated metallized film. This film is, in turn, placed in the center of a 10 cm x 17 cm sheet of nonwoven material, coated with an acrylic adhesive.

Approximately 600 mg (milligrams) of the gel composition made above is then placed on the lens tissue, and spread evenly. The patch is then applied to the skin of a human subject, experiencing post-operative pain, with the gel/tissue layer in direct contact with the skin. The subject's pain is reduced.

In the above example, other transdermal patch structures may be substituted for the patch structure described, with substantially similar results. Many structures useable herein are among those known in the art. Also, in the above example, methyl caproate, propyl caproate, hexyl acetate, methyl heptylate, pentyl heptylate, heptyl acetate, heptyl caproate, propyl caprylate, octyl acetate, octyl butylate, methyl caprate, ethyl caprate, hexyl caprate, methyl pelargonate, butyl pelargonate, lauryl acetate, lauryl butylate, methyl laurate, ethyl laurate, isopropyl laurate, hexyl laurate, $C_{12}$-$C_{15}$ alcohol lactate, methyl myristate, ethyl myristate, isopropyl myristate, dimethyl adipate, diisopropyl adipate, diisobutyl adipate, dimethyl maleate, diisopropyl malate, dibutyl malate, dihexyl maleate, and mixtures thereof are substituted for methyl caprylate, with substantially similar results.


## EXAMPLE II


A buprenorphine composition, according to this invention, is made comprising:

| Component | % (by weight) |
|---|---|
| buprenorphine | 3.00 |
| citric acid | 0.96 |
| propylene glycol | 83.70 |
| lauryl alcohol | 1.92 |
| distilled water | 9.62 |
| hydroxypropylcellulose | 0.80 |

The composition is made by mixing the compounds in a manner analogous to the method of Example I. Approximately 400 milligrams of this composition are placed in a suitable patch structure having a surface area of approximately 5cm². When applied to the skin of a human subject, analgesia is produced.

In the above example, 1,2 butane diol, glycerol and 1,2,6-hexane triol are substituted for propylene glycol, with substantially similar results. Also, in the above example, octanol, decanol, myristyl alcohol, myristoleyl alcohol, palmitoleyl alcohol, oleyl alcohol, linolenyl alcohol, linolyl alcohol, elaidyl alcohol, vaccenyl alcohol, petroselinyl alcohol, and petroselaidyl alcohol are substituted for lauryl alcohol, with substantially similar results.


## EXAMPLE III


A buprenorphine composition, according to this invention, is made comprising:

| Component | % (by weight) |
|---|---|
| buprenorphine | 3.00 |
| lactic acid | 1.20 |
| propylene glycol | 80.90 |
| methyl caprylate | 2.50 |
| methyl caproate | 2.50 |
| octanol | 4.95 |
| dimethyl isosorbide | 4.95 |

The methyl caprylate, methyl caproate, octanol and dimethyl isosorbide are added, separately, to a flask. The propylene glycol is then added, and the mixture mixed until a homogenous solution is obtained. The buprenorphine and lactic acid are then added to the solvent solution. The mixture is sonicated for approximately 15 minutes, heated to approximately 50° C (122° F) for approximately 30 minutes, and then stirred for approximately hours at approximately 32° C (90° F). Approximately 0.5 ml of the composition is then placed in a patch structure, such as described in Example I. The patch, when applied to the skin of a human suffering pain, affords analgesia.

In the above example, acetic acid, propionic acid, citric acid, tartaric acid, malic acid, and maleic acid are substituted for lactic acid, with substantially similar results.


EXAMPLE IV


A buprenorphine composition, according to this invention, is made comprising:

| Component | % (by weight) |
|---|---|
| buprenorphine HCl | 3.0 |
| propylene glycol | 81.4 |
| oleic acid | 4.8 |
| distilled water | 10.0 |
| hydroxypropylcellulose | 0.8 |

The composition is made by mixing the components in a manner analogous to the method of Example I. Approximately 400 milligrams of this composition are placed in a suitable patch structure having a surface area of approximately 10cm². When applied to the skin of a human subject, analgesia is produced.

In the above example, caprylic acid, capric acid, lauric acid, myristic acid, linolenic acid, and linoleic acid are substituted for oleic acid with substantially similar results.


EXAMPLE V


A buprenorphine ointment, according to this invention, is made comprising:

| Component | % (by weight) |
|---|---|
| buprenorphine HCl | 2.00 |
| 1,2-butane diol | 78.50 |
| $C_{12}$-$C_{15}$ alcohols lactate | 9.0 |
| Brij 721* | 10.0 |
| fragrance | 0.5 |

*: polyoxyethylene 21 stearyl ether, nonionic emulsifier, sold by ICI Americas, Inc

The Brij 21 is melted, and mixed with a heated mixture of the 1,2-butane diol and $C_{12}$-$C_{15}$ alcohols lactate. The buprenorphine HCl is added and stirred, and the mixture is cooled. This composition, when applied to the skin of a human subject suffering pain from metastatic cancer, affords analgesia.

## Claims

1. A composition for the transdermal delivery of buprenorphine, comprising a safe and effective amount of a pharmaceutically-acceptable buprenorphine salt in a carrier comprising:
(a) a polar solvent material selected from the group consisting of $C_3$-$C_4$ diols, $C_3$-$C_6$ triols, and mixtures thereof; and
(b) a polar lipid material selected from the group consisting of fatty acids, fatty alcohols, fatty alcohol esters, fatty acid esters, and mixtures thereof;
wherein said polar solvent material and said polar lipid material are present in a weight ratio of solvent material:lipid material of from 60:40 to 99:1.

2. A composition for the transdermal delivery of buprenorphine, according to Claim 1, wherein said polar lipid material is selected from the group consisting of: $C_{12}$-$C_{18}$ fatty acids, $C_{12}$-$C_{18}$ fatty alcohols, monoesters of a $C_8$-$C_{12}$ fatty alcohol, monoesters of a $C_8$-$C_{12}$ fatty acid, diesters of $C_2$-$C_8$ dicarboxylic acids with $C_1$-$C_6$ alcohols, diesters of $C_2$-$C_8$ dihydroxy alcohols with $C_2$-$C_6$ acids, and mixtures thereof; preferably methyl caprylate, propyl caprylate, octyl acetate, methyl caproate, propyl caproate, methyl laurate, $C_{12}$-$C_{15}$ alcohols lactate, diisopropyl adipate, diisobutyl adipate, and mixtures thereof.

3. A composition for the transdermal delivery of buprenorphine, according to Claim 2, wherein said polar solvent material is selected from the group consisting of: 1,2-butane diol, 1,2-propane diol, glycerol and 1,2,6-hexane triol, and mixtures thereof; preferably 1,2-propane diol.

4. A composition for the transdermal delivery of buprenorphine, according to Claim 3, wherein said polar lipid material is methyl laurate, and the weight ratio of solvent material:lipid material is 97:3.

5. A composition for the transdermal delivery of buprenorphine, according to Claim 3, wherein said polar lipid material is methyl caprylate, and the weight ratio of solvent material:lipid material is from 90:10 to 95:5.

6. A composition for the transdermal delivery of buprenorphine, according to Claim 3, wherein said polar lipid material is selected from the group consisting of lauryl alcohol, myristyl alcohol, oleyl alcohol, lauric acid, oleic acid, linoleic acid and mixtures thereof.

7. A composition for the transdermal delivery of buprenorphine, according to Claim 1, wherein said buprenorphine salt comprises buprenorphine and a pharmaceutically-acceptable acid, preferably citric acid, lactic acid, and mixtures thereof.

8. A composition for the transdermal delivery of buprenorphine, according to Claim 2, additionally comprising a solvent material which enhances the solubility of said polar lipid material in said polar solvent material.

9. A composition for the transdermal delivery of buprenorphine, according to Claim 2, additionally comprising a thickener.

10. A composition in unit dosage form, for the transdermal delivery of buprenorphine, comprising:
(a) from 1.0 mg to 40 mg, preferably from 3.0 mg to 10 mg, of buprenorphine as a pharmaceutically-acceptable buprenorphine salt; and
(b) a carrier comprising
(i) propylene glycol, and

(ii) a polar lipid material selected from the group consisting of oleic acid, lauryl alcohol, myristyl alcohol, oleyl alcohol, methyl caprylate, propyl caprylate, octyl acetate, methyl caproate, propyl caproate, methyl laurate, $C_{12}$-$C_{15}$ alcohols lactate, diisopropyl adipate, diisobutyl adipate, and mixtures thereof; preferably methyl laurate, methyl caprylate, or lauryl alcohol;

wherein said polar solvent material and said polar lipid material are present at a combined level of from 70% to 99.5%, preferably from 80% to 99.5%; and wherein said polar solvent material and said polar lipid material are present in a weight ratio of solvent material:lipid material of from 60:40 to 99:1.